# EUROPEAN PATENT SPECIFICATION

(11) **EP 0 493 433 B1**
(45) Date of publication and mention of the grant of the patent: **10.01.1996**
(21) Application number: 90913763.0
(22) Date of filing: 19.09.1990
(51) Int. Cl.: C12P 21/08

(54) **METHOD FOR IMPROVING HUMAN MONOCLONAL ANTIBODY PRODUCTION AND CELLS USED THEREIN**
VERFAHREN ZUR VERBESSERTEN HERSTELLUNG VON MENSCHLICHEN ANTIKÖRPERN SOWIE HIERZU BENUTZTE ZELLEN
PROCEDE D'AMELIORATION DE LA PRODUCTION D'ANTICORPS MONOCLONAUX HUMAINS ET CELLULES UTILISEES DANS CE PROCEDE

(30) Priority: 19.09.1989 US 409621
(43) Date of publication of application: 08.07.1992
(73) Proprietor: CENTOCOR, INC., Malvern, PA 19355 (US)
(72) Inventor: KNIGHT, David, M., Paoli, PA 19301 (US); GHRAYEB, John, Thorndale, PA 19372 (US)
(74) Representative: Holdcroft, James Gerald, Dr.
(86) International application number: US9005322
(87) International publication number: WO9104336

(56) References cited:
- EP-A- 0 125 023
- EP-A- 0 173 494
- EP-A- 0 271 379
- EP-A- 0 314 161
- EP-A- 0 315 062
- EP-A- 0 328 404
- EP-A- 0 388 964
- Science, Vol. 229, September 1985 S. L. Morrison: "Transfectomas Provide Novel Chimeric Antibodies ", pp. 1202-1207
- Nature, Vol. 312, December 1984 G. L. Boulianne et al.: "Production of functional chimaeric mouse/human antibody ", pp. 643-646
- Nature, Vol. 312, December 1984 M. S. Neuberger et al.: "Recombinant antibodies possessing novel effector functions ", pp. 604-608
- Science, Vol. 229, August 1985 Jean L. Marx: "Antibodies Made to Order ", pp. 455-456
- National Library of Medicine, File Med 88, Accession no. 88297774, Ramachandra RN: "Human--Human hybrido-mas secreting lipid A reactive monoclonal antibodies", Immunol Lett 1988 Jun;18(2):93-7

## Description

### Background

The successful utilization of human monoclonal antibodies for treatment and diagnosis of human disease is potentially limited by difficulties associated with their commercial production. Although murine monoclonal antibodies are relatively easy to generate in commercial quantities, they have been associated with immunogenicity in humans which reduces their effectiveness in vivo. Development of an immune response against mouse proteins can neutralize the therapeutic effects of murine monoclonal antibodies and trigger potentially dangerous anaphylactic reactions.

Human monoclonal antibodies offer several advantages over murine monoclonal antibodies, particularly as potential pharmaceutical agents. For example, human monoclonal antibodies are not likely to provoke an immune response comparable to the human anti-mouse antibody (HAMA) response typically seen after administration of murine antibodies. A HAMA response may accelerate clearance of a circulating monoclonal antibody and block its binding to an antigen thereby reducing its effectiveness. Human monoclonal antibodies may be more effective in interacting with the human immune system to activate therapeutically useful effector functions, such as antibody-dependent cellular cytotoxicity, phagocytosis or complement fixation.

Human antibodies also represent a different repertoire of specificities, and therefore may recognize epitopes not detected by foreign antibodies. The effectiveness of human antibodies is less likely to be compromised by problems associated with immunogenicity such as immune complex formation or anaphylaxis.

In spite of the many advantages of human monoclonal antibodies for therapy, their potential has not been realized, primarily because technical obstacles have limited the number and quality of cell lines that secrete them. Human hybridoma technology suffers from a number of technical shortcomings. Hybrids formed by fusion of mouse myeloma cells with human lymphoid cells display a preferential loss of human chromosomes making it difficult to achieve long-term production of human monoclonal antibodies. The use of human myeloma cell lines as fusion partners has been successful in some cases but there remains a problem with long term stability. Human monoclonal antibodies are difficult to generate and produce in commercial quantities because of these problems. Antibodies that have been generated, either from totally human hybridomas or human-rodent heterohybridomas can be produced only at low levels. These hybridomas often suffer from low antibody production and instability of the antibody-producing phenotype, presumably due to loss of crucial chromosomes.

Clinical immunology research and in vivo therapy would benefit greatly if methods were available for reproducibly generating human monoclonal antibodies.

### Summary of the Invention

The present invention provides a method for producing selected human monoclonal antibodies using recombinant DNA technology and cells used to produce the antibodies. In this method, regions of human immunoglobulin genes, generally derived from a hybridoma, are cloned and expressed in a host cell. The method involves preparing a first nucleic acid sequence which encodes a human heavy chain of the selected monoclonal antibody and a human regulatory sequence, and a second nucleic acid sequence which encodes a human light chain of the selected monoclonal antibody and a human regulatory sequence. The first and second nucleic acid sequences are used to transform a host cell. The transformed host cells are cultured under conditions appropriate to selectively grow the transformed cells. Recombinant monoclonal antibodies produced by the transformed host cell are then recovered.

The recombinant monoclonal antibodies produced by the method are substantially identical to the original human monoclonal antibodies, i.e., the monoclonal antibodies produced by the hybridoma. Transformed host cells produced by the present method are stable over many generations, and produce higher quantities of the human monoclonal antibody than the original hybridomas.

The present method has several advantages, including improved production levels of the monoclonal antibody and stability of the transformed host cells. The antibody genes are stably integrated into the host cell genome and can be maintained in the transformed host cell, for example, by appropriate drug selection, resulting in a high level of expression of recombinant human monoclonal antibodies. The present method allows the monoclonal antibody isotype to be selected on a rational instead of a random basis. Thus, the isotype of the human monoclonal antibody can be chosen to maximize therapeutic effectiveness.

### Brief Description of th Figures

Figure 1 is a schematic representation of the DNA probes J_{H} and J_{K} used to screen the HA-1A library.

Figure 2 is a schematic representation of plasmids pSV2neoHuk(S) and pSV2gptHuk(S).

Figure 3 is a graph showing the results of a lipid A binding assay which shows that binding of the monoclonal antibody produced by the 148 cell line is indistinguishable from the binding of the original HA-1A C83 antibody.

Figures 4A and 4B are graphs showing the results of experiments comparing the C83 cell line with the 148 cell line for antibody production (4A) and cell number (4B).

Figure 5 is a schematic representation of plasmids pSV2gptC83DP and pSV2neoC83DP.

### Detailed Description of the Invention

In the method of the invention, nucleic acid sequences, or genes, are prepared which encode human heavy and light chains of a selected monoclonal antibody, and human regulatory nucleotide sequences specific for the genes. Alternatively, the first and second nucleic acid sequences can encode just the variable regions of the heavy and light chains. The first nucleic acid sequence encodes all or a portion or the heavy chain and its associated regulatory sequence, and the second nucleic acid sequence encodes all or a portion of the light chain and its associated regulatory sequence. The genes are generally derived from a hybridoma which produces the selected monoclonal antibody but can be obtained from other sources which contain the nucleotide sequences encoding the antibody proteins. The genes can be derived from different hybridomas, that is, the light chain sequence from one hybridoma and the heavy chain sequence from a different hybridoma. The hybridoma can be any human/human or human/murine hybridoma of choice which produces the selected human monoclonal antibody.

As used herein, the term "human/human hybridoma" means fused hybrid cell lines created by fusing a B lymphocyte with a long-lived neoplastic plasma cell, or a T lymphocyte, with a lymphoma cell, wherein the fusion partners of both are of human origin. A "human/ murine hybridoma" refers to fused hybrid cell lines wherein one of the fusion partners is of murine origin.

The expression "nucleic acid sequence" or "nucleotide sequence" refers to a linear segment or polymer of deoxyribonucleic acid (DNA) or ribonucleic acid (RNA). The polymer of DNA or RNA can be single- or double-stranded, optionally containing synthetic, non-natural, or altered nucleotides capable of incorporation into DNA or RNA polymers.

The first and second nucleotide sequences preferably include the regulatory nucleotide sequences which control the transcription and translation of the genes. The terms "human regulatory nucleotide sequence" or "human regulatory sequence", as used herein, refer to a nucleotide sequence of human origin which is located 5' and/or 3' to a nucleotide sequence the transcription of which is controlled by the human regulatory nucleotide sequence in conjunction with the protein synthetic apparatus of the cell. A human regulatory nucleotide sequence can include a promoter region, as that term is conventionally employed by those skilled in the art. A promoter region can include, for example, an association region recognized by an RNA polymerase, one or more regions which control the effectiveness of transcription initiation in response to physiological conditions, and the transcription initiation sequence and the regulatory elements required for expression of the gene (i.e., promoter, enhancer, octamer, splicing and polyadenylation signals).

In one embodiment of the present method, nucleic acid sequences encoding the intact heavy chains and light chains of the selected monoclonal antibody are isolated from a hybridoma by constructing a genomic library from the hybridoma DNA. The genomic library is.constructed by well-known techniques, e.g., utilizing a bacteriophage vector, such as lambda (λ) gt10 or λ EMBL-3. The vector is then used to infect a host cell, wherein the insert DNA is amplified. In a preferred embodiment of the present method, a genomic library is prepared from the hybridoma DNA and cloned into the bacteriophage vector λ EMBL-3.

The genomic library is screened or probed to determine which segments of the DNA in the library encode the heavy and light chains of the antibody. Heavy and light chain DNA probes are used to screen the genomic library. For example, DNA probes derived from the human light and heavy chain J regions of the immunoglobulin loci (hereinafter, J_{H} and J_{K} for heavy and light chains, respectively) can be used. Other probes from the immunoglobulin loci can be used, for example, constant region sequences from the heavy and light chain genes. Screening of the DNA library can be performed as described by Maniatis et al., Molecular Cloning : A Laboratory Manual, Cold Spring Harbor Laboratory, NY (1982).

DNA clones which have been identified as containing the desired human heavy and light chain genes are isolated from the genomic library. Restriction endonuclease maps of the clones are constructed and compared to published maps to verify that they are derived from the immunoglobulin loci. Ravetch et al., Cell, 27:583-591 (1981); Klobeck and Zachaw, Nucleic Acids Research, 14:4591-4603 (1986). The variable regions can be isolated and used as probes in Northern blot analysis (Maniatis et al., supra.) to verify that these sequences are expressed in the original hybridoma.

The isolated nucleic acid sequences are subcloned by standard techniques into vectors, e.g., plasmid or viral vectors. Intact heavy and light chain genes can be cloned into separate plasmids or combined into a single large plasmid. If a different isotype is desired, or if only the variable region is cloned for the heavy or light chain, the variable region alone is cloned into vectors containing previously cloned constant regions.

The regulatory elements required for expression of the antibody genes in the host cell (i.e., promotor, enhancer, octamer, splicing and polyadenylation signals) are generally present on the cloned DNA, as part of the regulatory sequences. In addition to the nucleic acid sequences encoding the heavy and light chains and associated regulatory sequences, the vector can also include a marker gene for selection in mammalian cells, which allows the host cells expressing the foreign DNA to grow in selective media. For example, the plasmid vectors pSV2gpt (ATCC No. 37145) or pSV2neo (ATCC No. 37149) are particulary useful in the present method.

Plasmid pSV2gpt contains a gene encoding a protein necessary to confer resistance to the drug mycophenolic acid, and pSV2neo contains a gene encoding a protein necessary to confer resistance to the drug G418. To allow growth in bacterial cells for ease of manipulation, an antibiotic resistance gene such as Amp^{r} (ampicillin resistance) can be included in the vector. Plasmids which are particularly useful are plasmids containing the human CK regions. Oi and Morrison, Biotechniques, 4:214-221 (1986).

The vectors containing the cloned antibody genes are transfected into a suitable host cell line by any known transfection method, e.g., electroporation, calcium phosphate precipitation, DEAE-dextran, or protoplast fusion. Any mammalian cell that supports high levels of synthesis and secretion of functional antibody molecules can be used as a host cell. A well characterized, non-producing murine myeloma cell line is particularly useful as a host. A preferred host cell line is a murine myeloma cell line which lacks any non-characterized human DNA, viral nucleic acids or viral particles. Utilizing a murine myeloma host cell reduces or eliminates the possibility of contamination of the human monoclonal antibody preparation with human viruses or uncharacterized human DNA, which could occur if a human host cell was used. Examples of suitable murine myeloma host cells include SP2/0-Ag14 and P3X63-Ag8,653, both of which are available from the American Type Culture Collection.

After transfection, the cells are grown in selective media such that only cells that have acquired the transfected DNA can survive. As previously stated, the plasmid vector contains a selectable marker gene encoding a protein which confers resistance to certain drugs on transformed cells containing the gene. Suitable selective drugs include G418, mycophenolic acid and hygromycin.

Cell clones that express or secrete antibody are identified, for example, by assaying the culture supernatant for the presence of antibody by any conventional assay technique, such as enzyme-linked immunosorbent assay (ELISA) or particle concentration fluorescence immunoassay. Monoclonal antibodies produced by the transformed cell line can be purified by conventional techniques, such as protein A affinity chromatography or standard biochemical procedures.

In one embodiment of the present invention, a library of genomic DNA fragments was constructed from the DNA of a human-derived hybridoma which produces a human monoclonal antibody specific for the lipid A portion of a bacterial gram-negative lipopolysaccharide. The genomic library was constructed in a bacteriophage vector, λ EMBL-3, using standard recombinant DNA techniques. The library was screened with a J region probe from the human kappa (κ) locus and a J region probe from the human heavy chain locus. These probes hybridized with the rearranged human light and heavy chain genes. Positive clones containing the variable regions of the heavy and light chains were isolated and characterized by restriction enzyme mapping.

The light chain variable region was cloned into a plasmid vector already containing the human κ region. The entire heavy chain gene, including variable and µ constant regions, was cloned into the resulting plasmid, so that functional antibody genes were recreated. Two such plasmids were constructed differing only in the selectable marker which was employed (i.e., gpt and neo) to ensure the presence of at least two copies of each gene (i.e., heavy and light chain genes) in the cell line.

The plasmids were transfected into a host murine cell line, SP2/0, by electroporation. The SP2/0 cell line was chosen to maximize antibody production and to minimize potential virus contamination of the product. This cell line grows well and has previously been used to express chimeric mouse-human antibodies. The cells were cultured in media containing drugs which selectively allow growth of cells expressing the drug-resistance genes, that is, cells which have incorporated and express the plasmid DNA. Drug-resistant clones were tested for the presence of secreted antibody in the cell supernatant. The clones producing the highest level of antibody were then subcloned, and after several subcloning cycles, a stable, high-level producer was selected.

The transfected cells were stable over many generations because the human immunoglobulin (Ig) genes become integrated into the host cell genome. Drug selection can be maintained, but is not always necessary, to ensure stability of the transformed cell lines. Some subclones maintain a high level of antibody production in the absence of drug selection.

The resulting antibody was then analyzed in the a lipid A binding assay to demonstrate its equivalence with the original human monoclonal antibody derived from the hybridoma. The binding activity of the recombinant human monoclonal antibodies produced by the transformed cells was indistinguishable from the binding activity of antibodies produced by the original hybridoma.

Monoclonal antibodies produced by the present method can be used for in vitro, clinical and/or diagnostic purposes, such as immunoassays. The present monoclonal antibodies are particularly useful for in vivo therapeutic purposes due to the increased quantities of uniform antibodies which are produced by the present method. For in vivo purposes, human monoclonal antibodies are superior to mouse immunoglobulins as they generally persist in the circulation for longer periods, and do not elicit a strong antibody response in the human. The present human recombinant monoclonal antibodies can be complexed with a drug or a cytotoxin or labeled with a radionuclide for therapeutic applications or a radionuclide for in vivo imaging of tumors, for example. Other applications include use in vaccines, or active immunization, e.g., with antiidiotypic antibodies to raise antibodies against pathogens not suitable for conventional vaccines and modulation of autoimmune and/or endocrine conditions with antireceptor antibodies.

The present invention is further illustrated by the following Exemplification.

### EXEMPLIFICATION

### Materials and Methods

### Cell Lines

HA-1A (cell line C83:MCB; M83-19, Centocor, Inc., Malvern, PA) is a mouse-human hybridoma generated by fusion of an EBV-transformed human splenic B-lymphocyte with a mouse-human hetero-myeloma. HA-1A secretes a human IgM,K antibody (referred to herein as "HA-1A antibody" or "HA-1A C83") with immunoreactivity towards the lipid A portion of bacterial gram-negative lipopolysaccharide. Growth medium for HA-1A was Iscove's medium supplemented with 5% fetal bovine serum (FBS). SP2/0 cells were obtained from the American Type Culture Collection (ATCC #1581) and grown in Iscoves medium supplemented with 10% FBS.

### Hybridization Probes

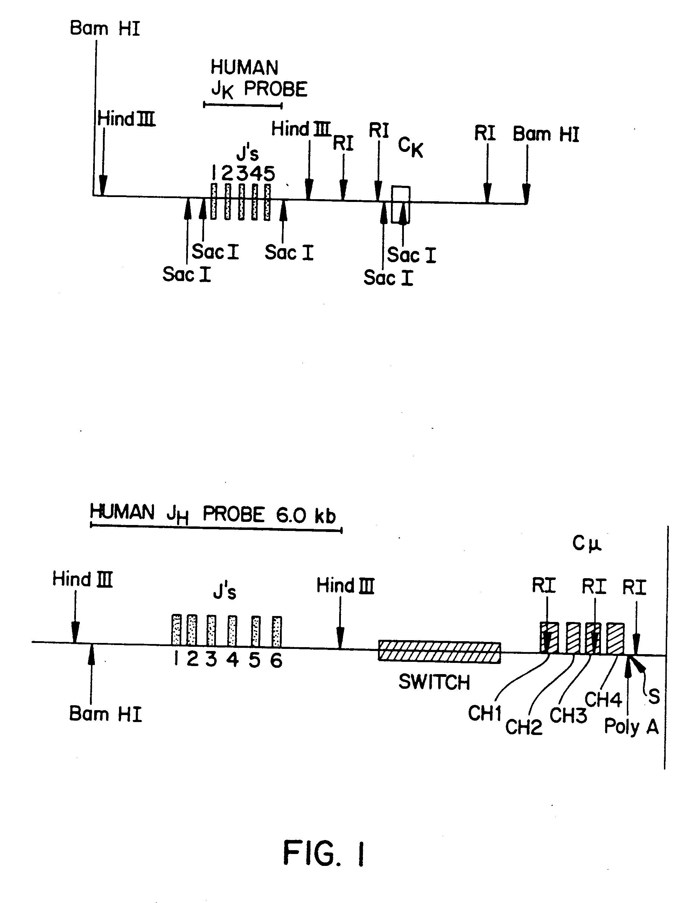

The DNA probes for screening the HA-1A library are shown schematically in Figure 1. The human heavy chain J_{H} probe is a 6.0 kb BamHI-HindIII fragment derived from the heavy chain locus containing all four J_{H} exons. The human light chain probe is a 1.8 kb SacI fragment from the kappa locus containing the five JK exons. ³P-labeled probes were prepared by nick translation using a kit obtained from Amersham, Inc. (Arlington Heights, IL). Unincorporated nucleotides were removed by centrifugation through a Sephadex ® G - 50 column. The specific activities of the probes were 5-10 x 10⁸ cpm/µg.

### Construction and Screening of a Genomic Expression Library

A genomic DNA library was constructed from HA-1A DNA from the cell line C83:MCB; M83-19 (hereinafter "C83"), a mouse/human heterohybridoma which produces a human anti-lipid A IgM monoclonal antibody. HA-1A genomic DNA was partially digested with restriction endonuclease Sau 3A and the resulting fragments were size-fractionated on a 10-40% sucrose density gradient. DNA fragments of approximately 15-23 kilobases (kb) were ligated to bacteriophage λ EMBL-3 arms previously digested with BamHI, and the resulting DNA was packaged in vitro into phage particles using Packagene (Promega Biotech, Inc., Madison, WI) according to the manufacturer's instructions. The phage packaged DNA was plated on 150 mm agar plates at a density of 30,000 plaques per plate.

The library was screened with the heavy chain and light chain J region ³P-labeled probes according to the method described by Maniatis et al., supra. Plaque hybridizations were carried out in 5X SSC, 50% formamide, 2X Denhardt's reagent, 200 µg/ml denatured salmon sperm DNA at 42°C for 18-20 hours. Final washes were in 0.5X SSC, 0.1% SDS at 65°C. Positive clones were identified after autoradiography and isolated after at least three rounds of plaque purification.

### DNA Sequencing

The clones were characterized by restriction mapping using standard methods as described by Maniatis et al., supra. Restriction endonuclease maps were determined for HA-1A V_{H} and V_{K} clones. Appropriate DNA fragments were subcloned into MP18 and MP19 and sequenced using the dideoxy method, with Sequenase (U.S. Biochemical Corp., Cleveland, OH) according to the manufacturer's instructions. The amino acid translations were compared with previously sequenced human light and heavy chains. A Northern blot analysis showed that the cloned V regions hybridized with the appropriate size RNAs from the original C83 cell line, demonstrating that the cloned sequences were expressed in C83.

### DNA Transfection using Electroporation

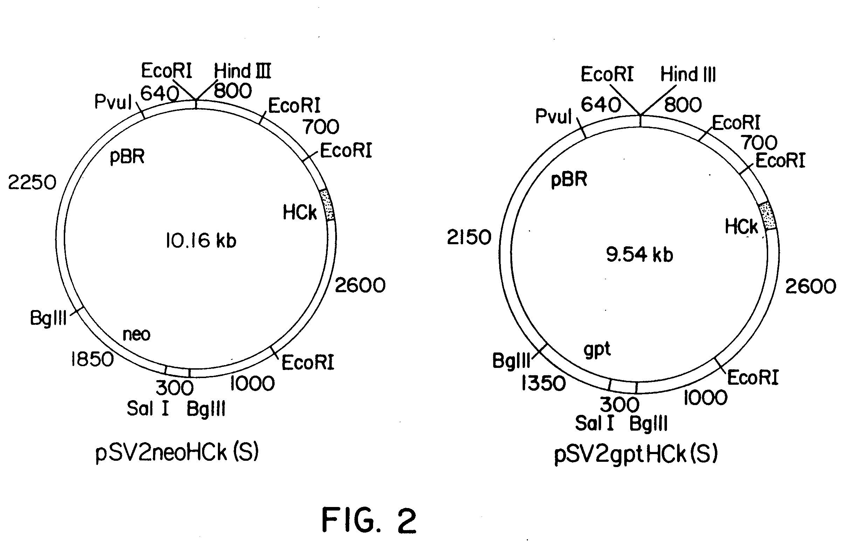

The light chain V region was then cloned into a plasmid expression vectors containing the human CK region and selectable markers, gpt, which confers resistance to the drug mycophenolic acid, and neo, which confers resistance to the drug G418. The starting plasmids, pSV2gptHCK (s) and pSV2neoHCK (s), are shown schematically in Figure 2. These plasmids were prepared from plasmids pSV2gpt (ATCC No. 37145) and pSV2neo (ATCC No. 37149) and a human CK region. The human CK regions were obtained from Sherie Morrison and are described by Oi and Morrison in Biotechniques, 4:214-221 (1986).

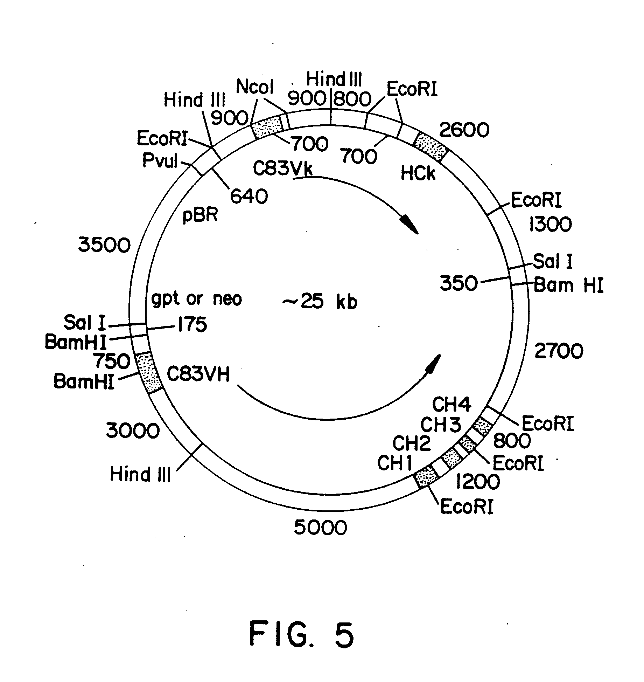

A second clone was isolated that contained an entire rearranged human heavy chain gene of the IgM type. Restriction enzyme mapping and DNA sequencing confirmed its similarity to other human antibody sequences. In addition, the variable region (V) sequence was shown to be expressed in the C83 cell line by Northern blot analysis. This gene was subcloned into the expression vectors already containing the light chain gene segments, resulting in two plasmids, pSV2gptC83DP and pSV2neoC83DP, with the potential for expressing both heavy and light chain genes derived from C83. A schematic representation of the plasmids is shown in Figure 5.

Plasmid DNA to be transfected was purified by centrifugation to equilibrium in ethidium bromide/cesium chloride gradients two times. 10-50 µg of plasmid DNA was added to 10⁷ SP2/0 cells in phosphate buffered saline (PBS) on ice, and the mixture was placed in a Biorad electroporation apparatus. (BioRad Laboratories, Richmond, CA) Electroporation was performed at 200 volts and the cells were plated out in 96 well microtiter plates. Drug selection was applied after 48 hours and drug resistant colonies were identified after 1-2 weeks. Selective medium included 0.25 µg/ml mycophenolic acid, 1.25 µg/ml hypoxanthine, 25 µg/ml xanthine, and 0.5 mg/ml G418.

### Quantitation of Antibody Production

Tissue culture supernatant was analyzed for human IgM protein content by ELISA assay using goat anti-human IgM Fc5u antibody (Jackson Laboratories, Avondale, PA). Human IgM (Jackson Laboratories) was used to generate a standard curve.

### Partial Purification of Recombinant HA-1A Antibodies

The recombinant antibody was purified by addition of 4% (w/v) polyethylene glycol (MW 6000) to clarified cell supernatant. After gentle mixing overnight at 4°C, the precipitate was collected by low speed centrifugation and solubilized in 0.05 M Tris/0.2 M NaC1/0.1M glycine, pH 8.6. The sample was then dialyzed against 0.3 M NaC1, 0.01 M sodium phosphate, pH 7.2.

### Lipid A Binding Assay

Lipid A binding activity was determined using a solid phase ELISA assay with monophosphoryl lipid A from S. minnesota R595 (RIBI Immunochem Research, Inc., Hamilton, MT) coated on microtiter plates and an alkaline phosphatase-conjugated goat anti-human IgM antibody (Jackson Laboratories).

### RESULTS

### Cloning of the HA-1A Heavy and Light Chain Genes

Several positive clones were isolated from an HA-1A genomic λ EMBL-3 library using either heavy or light chain probes. Following at least three rounds of plaque purification, bacteriophage DNA was isolated, digested with various restriction enzymes, and fractionated on 1% agarose gels to generate physical maps of the clones. The DNA was transferred to nitrocellulose and the blots were hybridized with J_{H} or J_{K} ³P-labeled probes to determine which fragments contained the adjacent variable regions. For the light chain, a 2.5 kb HindIII fragment was identified that hybridized to the J_{K} probe. This fragment was used as a ³P-labeled probe to verify that the V region sequence was expressed as RNA in the HA-1A hybridoma by Northern analysis. The 2.5 kb HindIII fragment was subsequently used for expression of the HA-1A light chain variable region. Restriction enzyme mapping indicated a heavy chain clone contained an entire rearranged µ heavy chain gene. This region was excised from the λ EMBL-3 phage using flanking SalI sites in the vector, and the 14 kb SalI fragment was used to express the HA-1A heavy chain.

### Sequence Analysis of HA-1A Variable Regions

The nucleotide sequences of the HA-1A antibody heavy and light chain variable regions were determined along with the deduced amino acid sequences, and were compared to the nucleotide sequences of the heavy and light chain variable regions of the recombinant 148 antibody. Comparison of the deduced HA-1A light chain sequence with human and mouse variable region sequence indicated that the light chain of HA-1A is a member of the human Vₖ IIIb subgroup, and is the result of joining of the V regions to the J_{K5} exon. The HA-1A heavy chain sequence reveals that it is a member of the human V_{H}II subgroup, and utilizes the human J6 exon. The deduced amino acid sequences of the 148 antibody are clearly human in character, and the first 95 amino acids of the light chain are identical to the sequence of a human IgM antibody, and the first 20 residues of the N-terminal amino acid sequences HA-1A antibody are identical to the seqeunce encoded in the recombinant light chain DNA.

### Construction of Expression Plasmids

The 2.5 kb HindIII fragment containing the putative HA-1A light chain variable region was cloned into the HindIII sites of plasmids pSV2gptHuk(S) and pSV2neoHuk(S), respectively. These plasmids contain the human CK region and dominant selectable markers gpt and neo, for selection in mammalian cells (Figure 2). The 14 kb SalI fragment containing the complete putative HA-1A heavy chain gene was cloned into the Sal I sites of the vectors containing the light chain variable region. The resulting plasmids, pSV2gpt283DP and pSV2neoC83DP, (shown schematically in Figure 5), differ only in the selectable marker employed, and contain single copies of both heavy and light chain genes. The expression plasmids were designed to direct expression of the antibody genes using the natural cis-acting regulatory sequences linked to the genes including the octamer sequences, promoters, enhancers, splice signals, and poly A addition signals.

To express the recombinant antibody, the two plasmids were cotransfected into the nonproducing murine myeloma-derived cell line SP2/0 and a double selection with mycophenolic acid and G418 was used to obtain stable transfectants. Two copies of each gene were transfected to augment the copy number in recipient cells, and to increase the likelihood of favorable integration sites. Resistant colonies were expanded and subclones were generated from the clones secreting the highest level of human IgM antibody as measured by ELISA assay. One subclone, designated cell line C83-148-3F3-14-21-31 (hereinafter, the "148" cell line or "C83/148"), was chosen for further study.

### Characterization of 148 Recombinant Antibody

HA-1A antibody is a pentameric IgM molecule. Proper assembly and secretion of pentameric IgM encoded by transfected genes requires elements not supplied by the heavy and light chain gene constructs themselves. To obtain pentameric recombinant IgM, such elements must be present in the recipient cell line. To determine whether the recombinant 148 antibody synthesized in SP2/0 cells is pentameric, purified 148 antibody was passed over an HPLC gel filtration column (Dupont Zorbax GF 450), and the elution profile compared to that of a standard human IgM pentameric antibody. The major 148 peak eluted at the same position as the standard (HA-1A) IgM indicating that the recombinant antibody is of a similar size to the standard.

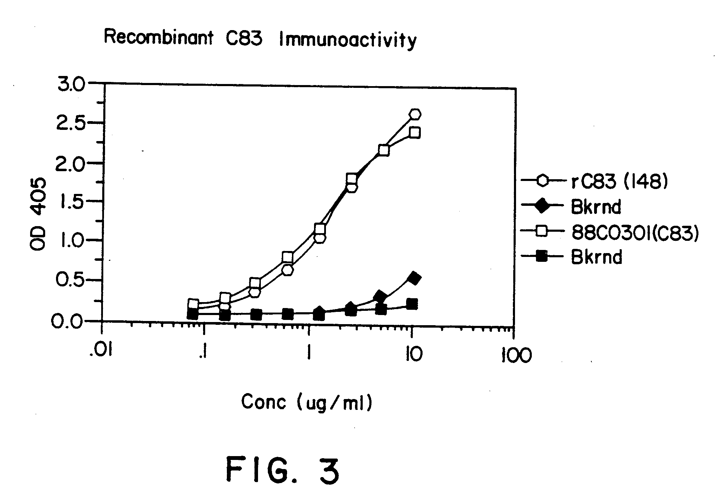

HA-1A antibody binds to the lipid A portion of the lipopolysaccharide (LPS) molecule derived from gram negative bacteria. To ascertain whether the 148 recombinant antibody retains the binding characteristics of HA-1A antibody, an immunoassay was performed using purified HA-1A from the original hybridoma, C83 (the C83 antibodies) and the 148 antibodies. Figure 3 shows the results of the lipid A binding assay. The binding curves for the two antibodies are indistinguishable, demonstrating that the 148 antibody (white circles) is equivalent to the C83 antibody (white squares) in the lipid A binding assay.

### Characteristics of Cell Line 148

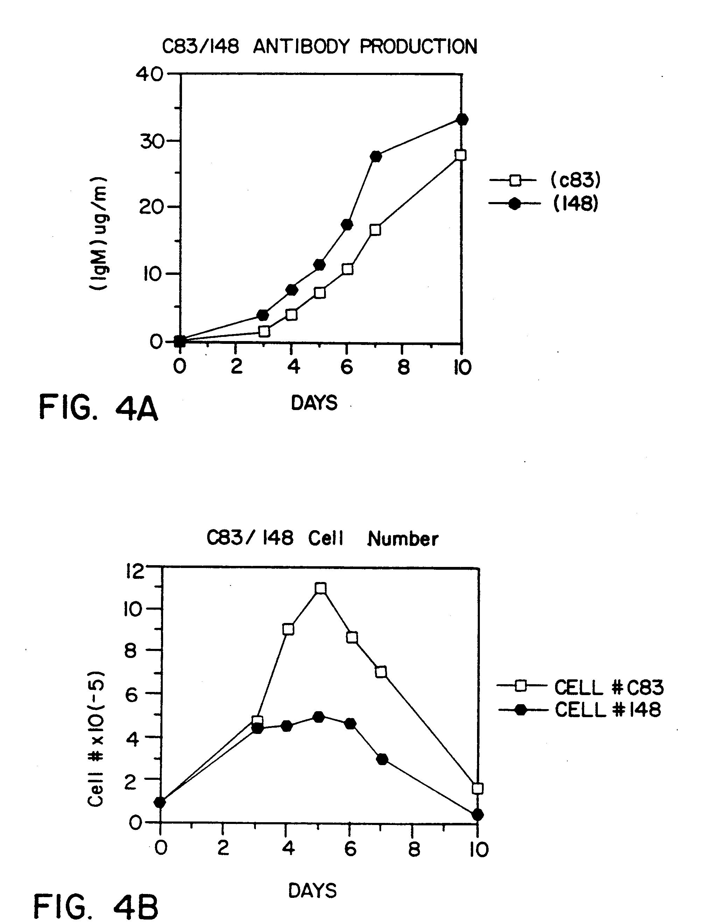

Figure 4 shows the results of an experiment in which the C83 cell line was compared to the 148 clone with regard to antibody production and cell number. The experiment was performed under growth conditions previously determined to be optimal for C83 (i.e., Iscoves's medium supplemented with 10% FBS). The amount of antibody produced by 148 and C83 are comparable (Figure 4A) even though 148 cells grow to a lower density under conditions optimized for C83 growth (Figure 4B). These results show that the 148 antibody production per cell is significantly higher than that of C83. At the 5 day point, when cell number is maximum for both cell lines, clone 148 produces 23 µg/ml/10⁶ cells whereas C83 produces 6.8 µg/ml/10⁶ cells. As higher 148 cell densities are achieved, production of the recombinant antibody would also be expected to increase.

To assess the stability of the antibody-producing phenotype for clone 148, the cells were cultured in the absence of the selective agents used during the initial selection after transfection. Antibody production decreased approximately 50% over the course of 60 days in the absence of selection. Subclones of clone 148 were obtained from the cultures that had been grown for 20 passages (approximately 60 days) without selection. A subclone, 148-35, exhibited growth and antibody production characteristics similar to 148 but is routinely cultured in the absence of selective agents. These data indicate that cell lines expressing transfected antibody genes can be obtained that do not require continued selective pressure to maintain high levels of antibody production.

### DISCUSSION

The present method demonstrates the feasibility of stabilizing and improving expression of human monoclonal antibodies by cloning the antibody genes, e.g., from a hybridoma, and expressing them in a new environment suitable for long term expression. In the embodiment illustrated in the Exemplification, a mouse-human heterohybridoma, HA-1A (C83), was used as the source of human antibody genes. The heavy and light chain antibody genes were cloned using human J region probes to screen genomic libraries made from HA-1A. The HA-1A light chain variable region was assembled with a previously cloned human kappa constant region and the entire HA-1A heavy chain gene into expression vectors containing selectable marker genes for mammalian cells. Although in this case the entire heavy chain gene was cloned, it is possible to use just the heavy chain variable region to assemble with a previously cloned constant region of the desired isotype to yield an antibody with the desired characteristics. Appropriate selection of the isotype can be useful to maximize the therapeutic effectiveness of the antibody. For example, the human IgG₁ isotype has been shown to be the most effective human isotype in mediating ADCC killing of human tumor cells. A cancer therapy monoclonal antibody, therefore, might be more effective when in the form of an IgG₁.

The transcriptional units in the expression plasmids utilized the natural human regulatory signals such as promotors, enhancers, and polyadenylation sequences. After transfection of the expression plasmids into the mouse myeloma cell line SP2/0, a cell line was isolated, designated 148, that secreted human IgM as determined by ELISA assay.

The antibody secreted by clone 148 appears to be equivalent to the HA-1A antibody secreted by the C83 hybridoma. The deduced amino acid sequences from the cloned DNA are clearly human in character when compared to other mouse and human antibody sequences. The first 95 amino acids of the light chain are identical to the sequence of a human IgM,K rheumatoid factor, although a different J exon is utilized. F. Goni et al., J. Immunol., 135:4073-4079 (1983). Light chains of this subgroup (V_{K}IIIb) comprise approximately 15% of normal Ig. D.K. Ledford et al., J. Immunol., 131:1322-1325 (1983). A partial N-terminal amino acid sequence has been performed on the HA-1A antibody and the first 20 residues are identical to the sequence encoded in the cloned light chain DNA.

Purified recombinant 148 antibody reacted with an anti-human IgM antibody in an ELISA assay, and was indistinguishable from the original HA-1A antibody in immunoreactivity towards lipid A. The recombinant antibody appears to be the correct size for pentameric IgM as determined by HPLC gel filtration chromatography.

The successful synthesis of a human antibody in a murine cell line demonstrates that there is no inherent species-specific barrier to efficient expression of human immunoglobulin promotors and enhancers can function in murine cells. Conversely, murine SP2/0 cells contain all the necessary factors in addition to synthetic and secretory apparatus to support the expression of exogenously supplied human antibody genes. Although this cross-species compatability might have been predicted, a high degree of sequence conservation between the immunoglobulin control regions of different species does not guarantee functional equivalence. For example, the rabbit kappa enhancer region is non-functional in mouse myeloma cells despite extensive sequence homology.

The 148 cell line that secretes the recombinant antibody has characteristics desirable for large scale antibody production. Overall IgM secretion levels are comparable to the original hybridoma and in fact exceed those of the hybridoma on a per cell basis. Although 148 antibody secretion gradually decreases in the absence of selective pressure, a subclone of 148 was obtained which produces high levels of antibody in the absence of selection for at least 60 days suggesting that large scale long term production can be achieved without the addition of expensive selective agents. The 148 cell line has also been adapted to growth in 10-fold lower fetal bovine serum than the original hybridoma while still maintaining high antibody secretion. The properties of this cell line demonstrate that the problems of low secretion and loss of production in the absence of selection often seen with expression of transfected antibody genes can be overcome.

The characteristics of the 148 cell line show that human antibody genes can be rescued from "problem" hybridomas and expressed in a format suitable for high level long-term economical antibody production. Cloning of the genes may be successful even in cases where they are present in amounts fewer than one copy per cell, e.g., if human chromosomes are being lost soon after fusion. To rescue these genes, either more plaques could be screened, or the human variable region sequences could be amplified via polymerase chain reaction prior to cloning. The recipient cell line can be chosen for its desirable characteristics, for example favorable growth kinetics, type of growth medium, and lack of endogenous pathogens or other undesirable contaminants. While avoiding the problems of low production and instability commonly associated with human-derived hybridomas, this approach also offers the advantage of a rational choice of monoclonal antibody isotype to maximize the effectiveness of a therapeutic antibody.

## Claims

1. A method for producing a selected human monoclonal antibody, comprising the steps of:
(a) preparing a first nucleic acid sequence encoding all or a portion of a human heavy chain of a human monoclonal antibody and a human regulatory sequence, and a second nucleic acid sequence encoding all or a portion of a human light chain of the selected human monoclonal antibody and a human regulatory sequence:
(b) transforming a mammalian host cell with said first and second nucleic acid sequences;
(c) culturing said transformed host cell; and
(d) recovering monoclonal antibody produced by said cultured host cell.

2. A method of claim 1, wherein the first and second nucleic acid sequences are both derived from a hybridoma which produces the selected human monoclonal antibody.

3. A method of claim 2, wherein (a) the first and second nucleic acid sequences are derived from a human/human or human/murine hybridoma; or (b) the hybridoma is a mouse/human hybridoma designated HA-1A, generated by fusion of an EBV-transformed human splenic B-lymphocyte with a mouse-human hetero-myeloma, which secretes a human IgM,K antibody with immunoreactivity towards the lipid A portion of bacterial gram-negative lipopoly-saccharide, wherein the growth medium for HA-1A was Iscove's medium supplemented with 5% fetal bovine serum (FBS).

4. A method of claim 1, wherein (a) the first and second nucleic acid sequences are derived from different human-antibody-producing hybridomas; or (b) the first nucleic acid sequence encodes a variable region of the heavy chain of the monoclonal antibody, and the second nucleic acid sequence encodes a variable region of the light chain of the monoclonal antibody; or (c) the first nucleic acid sequence encodes an intact region of the heavy chain of the monoclonal antibody, and the second nucleic acid sequence encodes an intact light chain of the monoclonal antibody; or (d) the mammalian host cell comprises a murine myeloma cell, e.g. comprising ATCC CRL 1581 or ATCC CRL 1580; or (e) the human monoclonal antibody comprises an anti-lipid A immunoglobulin M antibody.

5. A method for producing recombinant human monoclonal antibodies comprising the steps of:
(a) preparing a first nucleic acid sequence encoding a variable region of a human heavy chain of the antibody and a human regulatory sequence, and a second nucleic acid sequence encoding a variable region of a human light chain of the antibody and a human regulatory sequence;
(b) inserting the first and second nucleotide sequences into at least one expression vector;
(c) transfecting a mammalian host cell with the expression vector thereby transforming the host cells;
(d) selectively growing the transformed host cells; and
(e) recovering human monoclonal antibodies produced by the transformed host cells.

6. A method of claim 5, wherein the first and second nucleic acid sequences are derived from a hybridoma which produces the human monoclonal antibody.

7. A method of claim 6, wherein the hybridoma is a human/human or a human/murine hybridoma, e.g. a murine/human hybridoma designated HA-1A, generated by fusion of an EBV-transformed human splenic B-lymphocyte with a mouse-human hetero-myeloma, which secretes a human IgM,K antibody with immunoreactivity towards the lipid A portion of bacterial gram-negative lipopoly-saccharide, wherein the growth medium for HA-1A was Iscove's medium supplemented with 5% fetal bovine serum (FBS).

8. A method of claim 5, wherein (a) the first nucleotide sequence encodes an intact human heavy chain of the antibody; or (b) the second nucleotide sequence encodes an intact human light chain of the antibody.

9. A method of claim 5, wherein the expression vector is a plasmid.

10. A method of claim 9, wherein (a) the plasmid vector contains a nucleotide sequence encoding a human constant region of a human light chain of the antibody; or (b) the plasmid vector contains a nucleotide sequence encoding a constant region of a human heavy chain of the antibody; or (c) the plasmid vector contains a selectable marker gene.

11. A method of claim 9, wherein (a) the selectable marker gene comprises gpt or neo; or (b) the plasmid comprises the vector of Figure 5, wherein the selectable marker is gpt; or (c) the plasmid comprises the vector of Figure 5, wherein the selectable marker is neo.

12. A method of claim 5, wherein the host cell comprises a murine myeloma cell, comprising e.g. ATCC CRL 1581 or ATCC CRL 1580.

13. A method of claim 5, wherein the human monoclonal antibody comprises an anti-lipid A immunoglobulin M antibody.

14. A murine myeloma cell transformed with the plasmid of Figure 5 having the selectable marker gpt, or neo.

15. Cell line ATCC CRL 1581 cotransfected with the plasmids of Figure 5; or a recombinant human monoclonal antibody produced by said cell line.

16. A murine myeloma cell which expresses a human monoclonal antibody specific for lipid A of a gram negative bacterial endotoxin, wherein the murine myeloma cell is transformed by inserting a first nucleic acid sequence encoding all or a portion of a human heavy chain of a human monoclonal antibody and a human regulatory sequence, and a second nucleic acid sequence encoding all or a portion of a human light chain of the selected human monoclonal antibody and a human regulatory sequence into the murine myeloma cell.

## Patentansprüche

1. Ein Verfahren zur Herstellung eines selektierten menschlichen monoklonalen Antikörpers, das die Schritte umfaßt:
(a) Herstellen einer ersten Nucleinsäure-Sequenz, die eine ganze oder einen Teil einer menschlichen schweren Kette eines menschlichen monoklonalen Antikörpers und eine menschliche regulatorische Sequenz codiert, und einer zweiten Nucleinsäure-Sequenz, die eine ganze oder einen Teil einer menschlichen leichten Kette des selektierten menschlichen monoklonalen Antikörpers und eine menschliche regulatorische Sequenz codiert;
(b) Transformieren einer Säugerwirtszelle mit besagter ersten und zweiten Nucleinsäure-Sequenz;
(c) Kultivieren besagter transformierten Wirtszelle; und
(d) Wiedergewinnen des von besagter kultivierten Wirtszelle produzierten monoklonalen Antikörpers.

2. Ein Verfahren gemäß Anspruch 1, worin die erste und zweite Nucleinsäure-Sequenz von einem Hybridom stammen, das den selektierten menschlichen monoklonalen Antikörper produziert.

3. Ein Verfahren gemäß Anspruch 2, worin (a) die erste und zweite Nucleinsäure-Sequenz von einem menschlichen/menschlichen oder menschlichen/murinen Hybridom stammen; oder (b) das Hybridom ein als HA-1A bezeichnetes Maus/Mensch Hybridom ist, das durch Fusion eines EBV-transformierten menschlichen B-Lymphocyten der Milz mit einem Maus-Mensch Heteromyelom gebildet wird, das einen menschlichen IgM,K Antikörper mit einer Immunoreaktivität gegen den Lipid A Teil des Lipopoly-Saccharids gram-negativer Bakterien sezerniert, und worin das Wachstumsmedium für HA-1A Iscoves Medium, supplementiert mit 5% fötalem Rinderserum (FBS), war.

4. Ein Verfahren gemäß Anspruch 1, worin (a) die erste und zweite Nucleinsäure-Sequenz von verschiedenen, menschliche Antikörper produzierenden Hybridomen stammen; oder (b) die erste Nucleinsäure-Sequenz eine variable Region der schweren Kette des monoklonalen Antikörpers codiert, und die zweite Nucleinsäure-Sequenz eine variable Region der leichten Kette des monoklonalen Antikörpers codiert; oder (c) die erste Nucleinsaure-Sequenz eine intakte Region der schweren Kette des monoklonalen Antikörpers codiert, und die zweite Nucleinsäure-Sequenz eine intakte leichte Kette des monoklonalen Antikörpers codiert; oder (d) die Saugerwirtszelle eine murine Myelomzelle umfaßt, z. B. ATCC CRL 1581 oder ATCC CRL 1580 umfaßt; oder (e) der menschliche monoklonale Antikörper ein Anti-Lipid A Immunoglobulin M Antikörper umfaßt.

5. Ein Verfahren zur Herstellung rekombinanter menschlicher monoklonaler Antikörper, das die Schritte umfaßt :
(a) Herstellen einer ersten Nucleinsäure-Sequenz, die eine variable Region einer menschlichen schweren Kette des Antikörpers und eine menschliche regulatorische Sequenz codiert, und einer zweiten Nucleinsäure-Sequenz, die eine variable Region einer menschlichen leichten Kette des Antikörpers und eine menschliche regulatorische Sequenz codiert;
(b) Inserieren der ersten und zweiten Nucleinsäure-Sequenz in mindestens einen Expressionsvektor;
(c) Transfektieren einer Säugerwirtszelle mit dem Expressionsvektor wodurch die Wirtszellen transformiert werden;
(d) Selektives Wachstum der transformierten Wirtszellen; und
(d) Wiedergewinnen der von transformierten Wirtszellen produzierten menschlichen monoklonalen Antikörper.

6. Ein Verfahren gemäß Anspruch 5, worin die erste und zweite Nucleinsäure-Sequenz von einem Hybridom stammen, das den menschlichen monoklonalen Antikörper produziert.

7. Ein Verfahren gemäß Anspruch 6, worin das Hybridom ein menschliches/menschliches oder ein menschli-ches/murines Hybridom ist, z. B. ein als HA-1A bezeichnetes murines/menschliches Hybridom, das durch Fusion eines EBV-transformierten menschlichen B-Lymphocyten der Milz mit einem Maus-Mensch Heteromyelom gebildet wird, das einen menschlichen IgM,K Antikörper mit einer Immunoreaktivität gegen den Lipid A Teil des Lipopoly-Saccharids gram-negativer Bakterien sezerniert, und worin das Wachstumsmedium für HA-1A Iscoves Medium, supplementiert mit 5% fötalem Rinderserum (FBS), war.

8. Ein Verfahren gemäß Anspruch 5, worin (a) die erste Nucleotid-Sequenz eine intakte menschliche schwere Kette des Antikörpers codiert; oder (b) die zweite Nucleotid-Sequenz eine intakte menschliche leichte Kette des Antikörpers codiert.

9. Ein Verfahren gemäß Anspruch 5, worin der Expressionsvektor ein Plasmid ist.

10. Ein Verfahren gemäß Anspruch 9, worin (a) der Plasmidvektor eine Nucleotid-Sequenz enthält, die eine menschliche konstante Region einer menschlichen leichten Kette des Antikörpers codiert; oder (b) der Plasmidvektor eine Nucleotid-Sequenz enthält, die eine konstante Region einer menschlichen schweren Kette des Antikörpers codiert; oder (c) der Plasmidvektor ein selektierbares Markergen enthält.

11. Ein Verfahren gemäß Anspruch 9, worin (a) das selektierbare Markergen gpt oder neo umfaßt; oder (b) das Plasmid den Vektor der Abbildung 5 umfaßt, worin das selektierbare Markergen gpt ist; oder (c) das Plasmid den Vektor der Abbildung 5 umfaßt, worin das selektierbare Markergen neo ist.

12. Ein Verfahren gemäß Anspruch 5, worin die wirtszelle eine murine Myelomzelle umfaßt, die beispielsweise ATCC CRL 1581 oder ATCC CRL 1580 umfaßt.

13. Ein Verfahren gemäß Anspruch 5, worin der menschliche monoklonale Antikörper einen Anti-Lipid A Immunoglobulin M Antikörper umfaßt.

14. Eine murine Myelomzelle, die mit dem Plasmid der Abbildung 5 mit dem selektierbaren Markergen gpt oder neo transformiert ist.

15. Zellinie ATCC CRL 1581, die mit den Plasmiden der Abbildung 5 cotransfiziert ist; oder ein von besagter Zellinie produzierter rekombinanter monoklonaler Antikörper.

16. Eine murine Myelomzelle, die einen menschlichen monoklonalen Antikörper exprimiert, der für Lipid A eines Endotoxins gram-negativer Bakterien spezifisch ist, worin die murine Myelomzelle durch Inserieren einer ersten Nucleinsäure-Sequenz, die eine ganze oder einen Teil einer menschlichen schweren Kette eines menschlichen monoklonalen Antikörpers und eine menschliche regulatorische Sequenz codiert, und einer zweiten Nucleinsäure-Sequenz, die eine ganze oder einen Teil einer menschlichen leichten Kette des selektierten menschlichen monoklonalen Antikörpers und eine menschliche regulatorischen Sequenz codiert, in die murine Myelomzelle transformiert ist.

## Revendications

1. Méthode pour la production d'un anticorps monoclonal humain sélectionné comprenant les étapes consistant à :
(a) préparer une première séquence d'acides nucléiques codant pour toute ou partie d'une chaîne lourde humaine d'un anticorps monoclonal humain et une séquence régulatrice humaine, et une seconde séquence d'acides nucléiques codant pour tout ou partie d'une chaîne légère humaine de l'anticorps monoclonal humain sélectionné et une séquence régulatrice humaine;
(b) transformer une cellule hôte de mammifère avec lesdites première et seconde séquences d'acides nucléiques ;
(c) cultiver ladite cellule hôte transformée; et
(d) récupérer l'anticorps monoclonal produit par ladite cellule hôte en culture.

2. Méthode selon la revendication 1, où la première et la seconde séquence d'acides nucléiques sont toutes deux dérivées d'un hybridome qui produit l'anticorps monoclonal humain sélectionné.

3. Méthode selon la revendication 2, où (a) la première et la seconde séquence d'acides nucléiques sont dérivées d'un hybridome homme/homme ou homme/souris; ou (b) l'hybridome est un hybridome souris/homme désigné par HA-1A, généré par la fusion de lymphocytes B spléniques humains transformés par l'EBV avec un hétéromyélome souris/homme, qui secrète un anticorps humain IGM,K avec une immuno-réactivité envers la partie lipide A du lipopolysaccharide d'une bactérie gram négative, où le milieu de croissance pour HA-1A est le milieu d'Iscove supplémenté en 5 % de sérum de bovin foetal (SBF).

4. Méthode selon la revendication 1, où (a) la première et la seconde séquence d'acides nucléiques sont dérivées de différents hybridomes produisant des anticorps humains ; ou (b) la première séquence d'acides nucléiques code pour une région variable de la chaîne lourde de l'anticorps monoclonal, et la seconde séquence d'acides nucléiques code pour la région variable de la chaîne légère de l'anticorps monoclonal ; ou (c) la première séquence d'acides nucléiques code pour une région intacte de la chaîne lourde de l'anticorps monoclonal, et la seconde séquence d'acides nucléiques code pour une chaîne légère intacte de l'anticorps monoclonal ; ou (d) la cellule hôte de mammifère comprend une cellule hôte de myélome murin dont, par exemple, les échantillons déposés à l'ATCC sous les numéros ATCC CRL 1581 ou ATCC CRL 1580; ou (e) l'anticorps monoclonal humain comprend un anticorps de type immunoglobuline M anti-lipide A.

5. Méthode pour la production d'anticorps monoclonaux humains recombinants comprenant les étapes consistant à :
(a) préparer une première séquence d'acides nucléiques codant pour une région variable d'une chaîne lourde humaine de l'anticorps et une séquence humaine régulatrice, et une seconde séquence d'acides nucléiques codant pour une région variable d'une chaîne légère humaine de l'anticorps et une séquence régulatrice humaine ;
(b) insérer la première et la seconde séquence nucléotidique dans au moins un vecteur d'expression ;
(c) transfecter une cellule hôte de mammifère avec le vecteur d'expression de façon à transformer les cellules hôtes ;
(d) faire sélectivement croître les cellules hôtes transformées ; et
(e) récupérer les anticorps monoclonaux humains produits par les cellules hôtes transformées.

6. Méthode selon la revendication 5, où la première et la seconde séquence d'acides nucléiques sont dérivées d'un hybridome qui produit l'anticorps monoclonal humain.

7. Méthode selon la revendication 6, où l'hybridome est un hybridome homme/homme ou homme/souris par ex. un hybridome souris/homme désigné par HA-1A généré par la fusion d'un lymphocyte B splénique humain transformé par l'EBV avec un hétéromyélome souris/homme, qui secrète un anticorps humain IGM,K avec une immuno réactivité envers la partie lipide A du lipopolysaccharide d'une bactérie gram négative, où le milieu de croissance pour HA-1A est le milieu d'Iscove supplémenté en 5 % de sérum de bovin foetal (SBF).

8. Méthode selon la revendication 5, où la première séquence nucléotidique code pour une chaîne lourde humaine intacte de l'anticorps ; ou (b) la seconde séquence nucléotidique code pour une chaîne légère humaine intacte de l'anticorps.

9. Méthode selon la revendication 5, où le vecteur d'expression est un plasmide.

10. Méthode selon la revendication 9, où (a) le plasmide vecteur contient une séquence nucléotidique codant pour une région constante humaine d'une chaîne légère humaine de l'anticorps ; ou (b) le plasmide vecteur contient une séquence nucléotidique codant pour une région constante d'une chaîne lourde humaine de l'anticorps ; ou (c) le plasmide vecteur contient un gène marqueur sélectionnable.

11. Méthode selon la revendication 9, où le gène marqueur sélectionnable comprend gpt ou neo ; ou (b) le plasmide comprend le vecteur de la figure 5, où le marqueur sélectionnable est gpt ; ou (c) le plasmide comprend le vecteur de la figure 5 où le marqueur sélectionnable est néo.

12. Méthode selon la revendication 5, où la cellule hôte comprend une cellule de myélome murin comprenant par exemple ATCC CRL 1581 ou ATCC CRL 1580.

13. Méthode selon la revendication 5 où l'anticorps monoclonal humain comprend un anticorps de type immunoglobuline M anti-lipide A.

14. Cellule de myélome murin transformée avec le plasmide de la figure 5 possédant les marqueurs sélectionnables gpt, ou néo.

15. Lignée cellulaire ATCC CRL 1581 cotrans-fectée avec les plasmides de la figure 5 ; ou un anticorps monoclonal humain recombinant produit par ladite lignée cellulaire.

16. Cellule de myélome murin qui exprime un anticorps monoclonal humain spécifique pour le lipide A de l'endotoxine d'une bactérie gram négative, où la cellule de myélome murin est transformée par l'insertion dans la cellule de myélome murin d'une première séquence d'acides nucléiques codant pour tout ou partie d'une chaîne lourde humaine d'un anticorps monoclonal humain et une séquence régulatrice humaine, et une seconde séquence d'acides nucléiques codant pour tout ou partie d'une chaîne légère humaine de l'anticorps monoclonal humain sélectionné et une séquence régulatrice humaine.
